# EUROPEAN PATENT SPECIFICATION

(11) **EP 3 731 847 B1**
(45) Date of publication and mention of the grant of the patent: **26.08.2026**
(21) Application number: 18895666.8
(22) Date of filing: 28.12.2018
(51) Int. Cl.: A61K 33/00, A61K 9/00, A61M 16/10, A61M 16/12, A61P 9/12, A61M 16/00

(54) **PULSED ADMINISTRATION OF INHALED NITRIC OXIDE FOR THE TREATMENT OF PULMONARY HYPERTENSION**
GEPULSTE VERABREICHUNG VON INHALIERTEM STICKOXID ZUR BEHANDLUNG VON PULMONALER HYPERTONIE
ADMINISTRATION PULSÉE D'OXYDE NITRIQUE INHALÉ POUR LE TRAITEMENT DE L'HYPERTENSION PULMONAIRE

(30) Priority: 28.12.2017 US 201762611331 P
(43) Date of publication of application: 04.11.2020
(73) Proprietor: Mallinckrodt Pharmaceuticals Ireland Limited, Dublin D15 TX2V (IE)
(72) Inventor: QUINN, Deborah, Morristown, New Jersey 07960 (US); SHAH, Parag, Morristown, New Jersey 07960 (US); DEKKER, Martin, Califon, New Jersey 07830 (US)
(74) Representative: FRKelly
(86) International application number: PCT/US2018/067794
(87) International publication number: WO 2019/133777

(56) References cited:
- US-A- 6 109 260
- US-A1- 2012 093 948
- US-A1- 2013 239 962
- US-A1- 2016 279 165
- US-A1- 2017 165 294
- HAJIAN BITA ET AL: "Pulmonary vascular effects of pulsed inhaled nitric oxide in COPD patients with pulmonary hypertension", INTERNATIONAL JOURNAL OF CHRONIC OBSTRUCTIVE PULMONARY DISEASE, vol. Volume 11, 5 July 2016 (2016-07-05), pages 1533 - 1541, XP055808573, DOI: 10.2147/COPD.S106480
- ANONYMOUS: "Bellerophon Therapeutics, Inc. 10-K Mar. 13, 2017 6:17 AM | Seeking Alpha", 13 March 2017 (2017-03-13), XP055808791, Retrieved from the Internet <URL:https://seekingalpha.com/filing/3457009> [retrieved on 20210528]
- ROWDEN ADAM: "What is a normal respiratory rate based on your age?", MEDICALNEWSTODAY, 4 January 2023 (2023-01-04), XP093074459, Retrieved from the Internet <URL:https://www.medicalnewstoday.com/articles/324409> [retrieved on 20230817]
- SIAFAKAS NM ET AL: "Optimal assessment and management of chronic obstructive pulmonary disease (COPD). The European Respiratory Society Task Force", EUROPEAN RESPIRATORY JOURNAL, vol. 8, no. 8, 1 August 1995 (1995-08-01), GB, pages 1398 - 1420, XP093074455, ISSN: 0903-1936, DOI: 10.1183/09031936.95.08081398

## Description

### TECHNICAL FIELD

Principles and embodiments of the present invention generally relate to the field of inhaled nitric oxide delivery.

### BACKGROUND

Inhaled nitric oxide (iNO) has been well established as an effective vasodilator for use in pediatric pulmonary hypertension such as persistent pulmonary hypertension of the newborn (PPHN). It has been proposed that iNO could be an effective vasodilator for the treatment of various types of pulmonary hypertension (PH), including pulmonary arterial hypertension (PAH) (WHO Group I), PH associated with left heart disease (WHO Group 2), PH associated with lung disease and/or chronic hypoxemia (WHO Group 3), chronic thromboembolic pulmonary hypertension (WHO Group 4) or PH with unclear multifactorial mechanisms (WHO Group 5).

US 2012/0093948 Al discloses portable and pure nitric oxide delivery systems which can be used to treat a variety of patient conditions.

US 2013/0239962 Al discloses methods of administering therapeutic gases comprising high concentrations of nitric oxide, particularly concentrations above 2000 ppm. Also described are methods of administering a therapeutic gas comprising nitric oxide to a patient, wherein a dose of nitric oxide is administered from a portable device that includes a delivery system and a minicylinder. Methods of intermittent administration of nitric oxide pulses are also described.

Hajian B, De Backer J, Vos W, Van Holsbeke C, Ferreira F, Quinn D, Hufkens A, Claes R, De Backer W. Pulmonary vascular effects of pulsed inhaled nitric oxide in COPD patients with pulmonary hypertension. Int J Chron Obstruct Pulmon Dis. 2016;11(1):1533-1541; https://doi.org/10.2147/COPD.S106480 discloses a proof-of-concept study to quantify pulmonary blood vessel caliber changes after iNO administration.

Siafakas NM et al discuss "Optimal assessment and management of chronic obstructive pulmonary disease (COPD). The European Respiratory Society Task Force", in EUROPEAN RESPIRATORY JOURNAL, vol. 8, no. 8, 1 August 1995 (1995-08-01), GB, pages 1398 - 1420, DOI: 10.1183/09031936.95.08081398

Accordingly, there is a need for new therapies that utilize iNO for the treatment of PH such as WHO Groups 1-5.

### SUMMARY

Provided is nitric oxide for use in the treatment of pulmonary hypertension, the use comprising:
administering a plurality of pulses of a gas comprising nitric oxide (NO) to a patient in need thereof over a plurality of breaths, wherein the gas comprising NO is not administered to the patient in at least one breath of the plurality of breaths, wherein the patient is administered a dose of NO in the range of about 5 to about 100 mcg/kg IBW/hr, and wherein:
(i) a maximum time period between successive pulses of the gas comprising NO does not exceed about 9 seconds, or
(ii) at least 400 pulses of the gas comprising NO is administered to the patient every hour.

Various aspects of the present invention pertain to NO for use in methods of treating PH by maintaining dosing frequency and/or minimizing skipped breaths during pulsed administration of iNO.

In one or more embodiments, the patient is administered an effective amount of iNO in combination with an effective amount of long-term oxygen therapy (LTOT).

In one or more embodiments, the iNO is administered to the patient during the first half of inspiration.

In one or more embodiments, the maximum number of consecutive skipped breaths does not exceed three, two or one breaths.

In one or more embodiments, the average number of consecutive skipped breaths does not exceed about 3, about 2.5, about 2, about 1.5, about 1 or about 0.5 breaths.

The effective amount of iNO is in the range of about 5 to about 100 mcg/kg IBW/hr, such as about 30 to about 75 mcg/kg IBW/hr.

In one or more embodiments, the iNO is administered for a certain minimum treatment time, such as about 10, about 15, about 20, about 30, about 40, about 50, about 60, about 70, about 80 or about 90 minutes, or about 1, about 2, about 3, about 4, about 5, about 6, about 7, about 8, about 9, about 10, about 11, about 12, about 16, about 18 or about 24 hours, or about 1, about 2, about 3, about 4, about 5, about 6 or about 7 days, or about 1, about 2, about 3, about 4, about 5, about 6, about 7 or about 8 weeks, or about 1, about 2, about 3, about 4, about 5, about 6, about 7, about 8, about 9, about 10, about 12, about 18 or about 24 months.

In one or more embodiments, the iNO is administered for a certain amount of time each day, such as at least about 1, about 2, about 3, about 4, about 5, about 6, about 7, about 8, about 9, about 10, about 11, about 12, about 16, about 18 or about 24 hours a day.

In one or more embodiments, the patient has a low, intermediate, or high probability of PH.

In one or more embodiments, the PH comprises one or more of PAH (WHO Group I), PH associated with left heart disease (WHO Group 2), PH associated with lung disease and/or chronic hypoxemia (WHO Group 3), chronic thromboembolic pulmonary hypertension (WHO Group 4) or PH with unclear multifactorial mechanisms (WHO Group 5).

In one or more embodiments, the patient has PAH.

In one or more embodiments, the patient has WHO Group 3 PH associated with interstitial lung disease (PH-ILD).

In one or more embodiments, the patient has WHO Group 3 PH associated with idiopathic pulmonary fibrosis (PH-IPF).

In one or more embodiments, the patient has WHO Group 3 PH associated with chronic obstructive pulmonary disease (PH-COPD).

In one or more embodiments, the patient has PH associated with pulmonary edema from high altitude sickness.

In one or more embodiments, the patient has PH associated with sarcoidosis.

In one or more embodiments, the patient has a ventilation-perfusion (V/Q) mismatch.

In one or more embodiments, the administration of iNO provides an average decrease in systolic pulmonary arterial pressure (sPAP) in a group of patients after at least 20 minutes of iNO administration of at least about 2 millimeters of mercury (mm Hg).

In one or more embodiments, the administration of iNO provides an average decrease in sPAP in a group of patients after 20 minutes of iNO administration of at least about 4 mm Hg.

### DETAILED DESCRIPTION

Before describing several exemplary embodiments of the invention, it is to be understood that the invention is not limited to the details of construction or process steps set forth in the following description. The invention is capable of other embodiments and of being practiced or being carried out in various ways.

It has surprisingly been discovered that the dosing frequency of iNO therapy has a substantial impact on the treatment efficacy in patients with PH. Accordingly, various aspects of the present invention pertain to NO for use in methods that maintain dosing frequency and/or minimize skipped breaths during pulsed administration of iNO.

In one or more embodiments, the patient or group of patients are diagnosed with PH. The patient(s) can be diagnosed by a cardiologist, pulmonologist or other physician according to suitable criteria using techniques such as echocardiography, right heart catheterization, etc. Examples of such criteria include, but are not limited to, patients that have a mean pulmonary arterial pressure (mPAP) at rest of at least 25 mm Hg, or a tricuspid regurgitation velocity greater than 2.9 m/s, or other combinations of factors as determined by an appropriate physician. The World Health Organization (WHO) has defined five categories of PH: PAH (WHO Group 1); PH associated with left heart disease (WHO Group 2), PH associated with lung disease and/or chronic hypoxemia (WHO Group 3), chronic thromboembolic pulmonary hypertension (WHO Group 4) or PH with unclear multifactorial mechanisms (WHO Group 5).

Examples of WHO Group 2 patients include those with systolic dysfunction, diastolic dysfunction and/or valvular disease.

Examples of WHO Group 3 patients include PH-COPD patients and those with interstitial lung disease (ILD) such as PH-IPF patients. Other examples of WHO Group 3 patients include those with combined pulmonary fibrosis and emphysema (CPFE), chronic high altitude exposure, or other lung diseases such as sleep disordered breathing or developmental diseases. COPD, ILD and other lung diseases can be diagnosed according to any suitable factor or combination of factors, such as those set forth in the guidelines of the American Thoracic Society. One exemplary set of criteria for diagnosing COPD is the Global initiative for chronic Obstructive Lung Disease (GOLD) criteria. In at least one embodiment, the patient has PH-COPD. In at least one embodiment, the patient has PH and ILD, such as a patient with PH-IPF. In at least one embodiment, the patient has PH associated with pulmonary edema from high altitude sickness.

In one or more embodiments, the patient or group of patients has a low, intermediate, or high probability of PH as determined by echocardiography or other suitable technique. One exemplary set of criteria for evaluating the probability of PH is set forth in the 2015 ESC/ERS Guidelines for Diagnosis and Treatment of Pulmonary Hypertension. In at least one embodiment, the patient has a low echocardiographic probability of PH. In at least one embodiment, the patient has a moderate echocardiographic probability of PH. In at least one embodiment, the patient has a high echocardiographic probability of PH.

Examples of WHO Group 5 patients include those with hematologic disorders, systemic disorders that have lung involvement (e.g. sarcoidosis, Langerhans cell histiocytosis, lymphangioleiomyomatosis, neurofibromatosis and vasculitis), metabolic disorders (e.g. thyroid disorders and glycogen storage disease), and other diseases such as tumor obstruction or renal failure. In at least one embodiment, the patient has PH associated with sarcoidosis.

In one or more embodiments, the patient has a V/Q mismatch.

The iNO may be administered by a series of pulses or any other suitable technique for delivering iNO to a patient's lungs. Exemplary devices for the administration of iNO are described in U.S. Pat. No. 5,558,083; U.S. Pat. No. 7,523,752; U.S. Pat. No. 8,757,148; U.S. Pat. No. 8,770,199; U.S. Pat. No. 8,893,717; U.S. Pat. No. 8,944,051; U.S. Pat. App. Pub. No. 2013/0239963; U.S. Pat. App. Pub. No. 2014/0000596; and U.S. Pat. App. Pub. No. 2016/0106949.

In one or more embodiments, iNO is administered by a NO delivery device utilizing cylinders containing NO and a carrier gas such as nitrogen (N₂). Exemplary NO cylinder concentrations include, but are not limited to, concentrations in the range of about 100 ppm to about 15,000 ppm, such as about 100, about 200, about 300, about 400, about 500, about 600, about 700, about 800, about 900, about 1000, about 1500, about 2000, about 2500, about 3000, about 3500, about 4000, about 4500, about 5000, about 6000, about 7000, about 8000, about 9000, about 10,000 or about 15,000 ppm. In one or more embodiments, the NO cylinder concentration is about 4880 ppm.

In one or more embodiments, the NO is generated bedside or at the point of administration. For example, various chemical reactions can be used to generate NO, such as reacting N₂ and oxygen (O₂) in the presence of an electrode, or reacting nitrogen dioxide (NO₂) with a reducing agent.

In one or more embodiments, the iNO is administered as a series of pulses. The iNO may have a specific pulse volume, such as about 0.1, about 0.2, about 0.3, about 0.4, about 0.5, about 0.6, about 0.7, about 0.8, about 0.9, about 1, about 1.5, about 2, about 3, about 4 or about 5 mL. The pulse volume may be the same from one breath to the next, or the pulse volume may vary according to the patient's breathing rate and/or the amount of iNO already delivered to the patient.

In one or more embodiments, the effective amount of iNO is in the range of about 5 to about 100 mcg/kg IBW/hr. A patient's ideal body weight correlates with the patient's estimated lung size, and is a function of the patient's sex and height. In various embodiments, the dose of iNO is about 5, about 10, about 15, about 20, about 25, about 30, about 35, about 40, about 45, about 50, about 55, about 60, about 65, about 70, about 75, about 80, about 85, about 90, about 95 or about 100 mcg/kg IBW/hr.

In one or more embodiments, a constant dose of iNO is delivered to the patient in each breath, such as a constant dose in nmol/breath, ng/breath or mL/breath. Exemplary doses include about 10, about 20, about 30, about 40, about 50, about 60, about 70, about 80, about 90, about 100, about 150, about 200, about 300, about 400, about 500, about 600, about 700, about 800, about 900, about 1,000 or about 1,500 nmol NO per breath.

In one or more embodiments, the iNO is administered at a constant concentration. For example, the iNO may be administered at a constant concentration of about 1 ppm to about 100 ppm. In various embodiments, the dose of iNO is about 1, about 2, about 3, about 4, about 5, about 10, about 15, about 20, about 25, about 30, about 35, about 40, about 45, about 50, about 55, about 60, about 65, about 70, about 75, about 80, about 85, about 90, about 95 or about 100 ppm.

In one or more embodiments, a desired quantity of gas is administered to the patient over a plurality of breaths in a way that is independent of the patient's respiratory pattern. For example, a patient's iNO dose may be prescribed in terms of mcg/kg IBW/hr, such that a desired amount is delivered to the patient every hour regardless of the patient's respiratory pattern or breathing rate. The NO delivery device may have an input such as a dial, display, touchscreen or other user interface to receive the patient's prescription. An amount of NO per breath (e.g. nmol NO, ng NO, mL of gas comprising NO, etc.) can be calculated based on the patient's current respiratory pattern, and that amount of NO can be delivered to the patient in the next breath or for several breaths. The NO delivery device may monitor the patient's respiratory pattern or breathing rate (or changes in the respiratory pattern or breathing rate) and re-calculate and/or otherwise adjust the amount of NO-containing gas that is delivered on the current breath or on subsequent breaths. The NO delivery device can have a control system with appropriate software and/or hardware (e.g. flow sensors, pressure sensors, processors, memory, etc.) for monitoring the breath, calculating or otherwise determining the amount of NO to be delivered, and be in communication with other components of the NO delivery device (e.g. flow sensors, pressure sensors, valves, gas conduits, etc.) for delivering the gas comprising NO. The amount of NO per breath can be calculated and/or adjusted after every breath or can be calculated and/or adjusted at certain intervals such as every minute, every 10 minutes, every 10 breaths, every 100 breaths, etc.

In one or more embodiments, the iNO is not delivered to the patient every breath and at least one breath is skipped during the iNO therapy. The time period between individual pulses of gas comprising NO can vary or can be constant. In various embodiments, a maximum time period between pulses, a maximum average time period between pulses and/or a minimum pulse frequency may be provided.

Various situations can result in iNO being skipped in a particular breath. For example, an intermittent dosing regimen may be utilized in which the iNO is administered every n^{th} breath, with n being greater than 1. In various embodiments, n is about 1.01, about 1.1, about 1.2, about 1.3, about 1.4, about 1.5, about 1.6, about 1.7, about 1.8, about 1.9, about 2, about 2.5, about 3, about 4, about 5, about 6, about 7, about 8, about 9 or about 10. When n is not a whole number (e.g. 1.1 or 2.5), n can represent an average over multiple breaths. As an example, administering iNO every 2.5 breaths indicates that iNO is administered an average of 2 breaths out of every 5 breaths (i.e. 5/2 = 2.5). Similarly, administering iNO every 1.1 breaths indicates that iNO is administered an average of 10 breaths out of every 11 breaths (i.e. 11/10 = 1.1). Similar calculations can be performed for other intermittent dosing regimens where iNO is administered every n^{th} breath, with n being greater than 1.

In one or more embodiments, an intermittent dosing regimen may be utilized in which predetermined breaths are skipped. The skipping of predetermined breaths can be based on predetermined patterns such as skipping every other breath, skipping every third breath, skipping two consecutive breaths and delivering on the third breath, etc. The predetermined pattern can include delivering gas comprising NO on every n^{th} breath, such as having n be greater than 1, for example about 1.01, about 1.1, about 1.2, about 1.3, about 1.4, about 1.5, about 1.6, about 1.7, about 1.8, about 1.9, about 2, about 2.5, about 3, about 4, about 5, about 6, about 7, about 8, about 9 or about 10.

In one or more embodiments, one or more breaths is skipped in a certain time period. For example, 1, 2, 3, 4, 5, etc. breaths may be skipped every hour, every 30 minutes, every 15 minutes, every 10 minutes, every minute, every 30 seconds, etc. In some embodiments, as little as one breath is skipped during the entire iNO therapy. In other embodiments, multiple breaths are skipped during iNO therapy.

In one or more embodiments, an intermittent dosing regimen may be utilized in which random breaths are skipped. The random breath skipping can be determined according to a random number generator and/or can be based on current clinical conditions such as the patient's respiratory pattern, the patient's breathing rate, the amount of iNO that has been delivered to the patient, the patient's iNO prescription, etc., and/or can be based on settings for the NO delivery device such as a minimum pulse volume.

In one or more embodiments, the NO delivery device may have a minimum quantity of gas that can be delivered in a breath, such as a minimum pulse volume. This minimum quantity of gas can be set by the user or can be a minimum threshold value set by the specifications of the NO delivery device. In one or more embodiments, when the quantity of gas comprising NO to be delivered to the patient in a particular breath is less than the minimum quantity of gas per breath (e.g. minimum pulse volume), administration of the gas is skipped for that breath. In one or more embodiments, when the breath is skipped, a new quantity of gas per breath is calculated and/or the quantity of gas is carried over and is added to the amount of gas to be delivered in one or more subsequent breaths.

In addition to the exemplary situations described above, other situations that can result in one or more breaths being skipped during iNO therapy are also encompassed by the present disclosure. Such situations include, but are not limited to, skipped breaths or a pause in iNO therapy due to: changing or switching the drug cylinder or cartridge; NO delivery device purging; engagement with other devices or delivery systems such as LTOT, continuous positive airway pressure (CPAP), bilevel positive airway pressure (BPAP), etc.; NO delivery device alarm conditions such as apnea, empty drug cylinder/cartridge, empty battery, etc.; or NO delivery device fault condition(s).

In one or more embodiments, there is a maximum time period between successive pulses of the gas comprising NO. For example, the time period between successive pulses may vary or may be constant, but an upper limit may be provided that prevents too long of a period between successive pulses of gas. In exemplary embodiments, the maximum time period between successive pulses of gas comprises NO does not exceed about 9, about 8.5, about 8, about 7.5, about 7, about 6.5 or about 6 seconds. In one or more embodiments, the maximum time period between successive pulses of the gas comprising NO is provided as a maximum number of breaths. In exemplary embodiments, the maximum number of consecutive skipped breaths does not exceed four, three, two or one breaths.

In one or more embodiments, the average time period between successive pulses of the gas comprising NO does not exceed a certain time period, such as not exceeding about 9, about 8.5, about 8, about 7.5, about 7, about 6.5 or about 6 seconds. Again, the time period between individual pulses can vary or can be the same.

In one or more embodiments, the average number of consecutive skipped breaths does not exceed about 3, about 2.5, about 2, about 1.5, about 1 or about 0.5 breaths.

In one or more embodiments, the frequency of pulse administration is provided as a number of pulses in a given time period, such as pulses per hour. For example, in one or more embodiments the patient is administered at least about 400, about 410, about 420, about 430, about 440, about 450, about 460, about 470, about 480, about 490, about 500, about 510, about 520, about 530, about 540, about 550, about 560, about 570, about 580, about 590, about 600, about 625, about 650, about 700, about 750, about 800, about 850, about 900, about 950 or about 1000 pulses of the gas comprising NO per hour.

Shorter durations may also be used, and these pulse frequencies can likewise be expressed in terms of pulses per minute or other time period. In one or more embodiments, the patient is administered at least about 6.6, about 6.7, about 6.8, about 6.9 about 7, about 7.1, about 7.2, about 7.3, about 7.4, about 7.5, about 7.6, about 7.7, about 7.8, about 7.9 about 8, about 8.1, about 8.2, about 8.3, about 8.4, about 8.5, about 8.6, about 8.7, about 8.8, about 8.9 about 9, about 9.5, about 10, about 10.5, about 11, about 11.5, about 12, about 12.5, about 13, about 13.5, about 14, about 14.5, about 15, about 16, about 17, about 18, about 19 or about 20 pulses per minute.

In one or more embodiments, the iNO is administered for a certain amount of time each day. For example, the iNO may be administered for at least about 1 hour a day. In various embodiments, the iNO is administered for at least about 1, about 2, about 3, about 4, about 5, about 6, about 7, about 8, about 9, about 10, about 11, about 12, about 16, about 18 or about 24 hours a day.

In one or more embodiments, the iNO is administered for a certain treatment time. For example, the iNO may be administered for at least about 10, about 15, about 20, about 30, about 40, about 50, about 60, about 70, about 80 or about 90 minutes, or about 1, about 2, about 3, about 4, about 5, about 6, about 7, about 8, about 9, about 10, about 11, about 12, about 16, about 18 or about 24 hours, or about 1, about 2, about 3, about 4, about 5, about 6 or about 7 days, or about 1, about 2, about 3, about 4, about 5, about 6, about 7 or about 8 weeks, or about 1, about 2, about 3, about 4, about 5, about 6, about 7, about 8, about 9, about 10, about 12, about 18 or about 24 months, or 1, 2, 3, 4 or 5 years.

In one or more embodiments, the patient is also receiving long-term oxygen therapy (LTOT). In various embodiments, the LTOT is administered for at least about 1, about 2, about 3, about 4, about 5, about 6, about 7, about 8, about 9, about 10, about 11, about 12, about 16, about 18 or about 24 hours a day. In various embodiments, the LTOT is administered at a dose of about 0.5 L/min to about 10 L/min, such as about 0.5, about 1, about 1.5, about 2, about 2.5, about 3, about 4, about 5, about 6, about 7, about 8, about 9 or about 10 L/min. The LTOT may be administered continuously or via pulses.

In one or more embodiments, the iNO therapy provides an average decrease in sPAP in a group of patients of at least about 1 mm Hg. In various embodiments, the average decrease in sPAP in the group of patients is at least about 1, about 1.5, about 2, about 2.5, about 3, about 3.5, about 4, about 4.1, about 4.2, about 4.3, about 4.4 or about 4.5 mm Hg.

In one or more embodiments, the iNO therapy decreases sPAP over a certain time period, such as after administering iNO for about 10, about 15, about 20, about 30, about 40, about 50, about 60, about 70, about 80 or about 90 minutes, or about 1, about 2, about 3, about 4, about 5, about 6, about 7, about 8, about 9, about 10, about 11, about 12, about 16, about 18 or about 24 hours, or about 1, about 2, about 3, about 4, about 5, about 6 or about 7 days, or about 1, about 2, about 3, about 4, about 5, about 6, about 7 or about 8 weeks, or about 1, about 2, about 3, about 4, about 5, about 6, about 7, about 8, about 9, about 10, about 12, about 18 or about 24 months.

In at least one embodiment, the administration of iNO provides an average decrease in sPAP in a group of patients after at least 20 minutes of iNO administration of at least about 1 mm Hg, such as at least about 1.5, about 2, about 2.5, about 3, about 3.5, about 4, about 4.1, about 4.2, about 4.3, about 4.4 or about 4.5 mm Hg.

### EXAMPLES

### Example 1 - Effect of Pulsed Administration iNO Therapy on sPAP in Patients with PH-COPD

This study was a Phase 2, placebo-controlled, double-blind, randomized, two-part, dose-confirming clinical study characterizing the pharmacodynamic effects of pulsed iNO versus placebo in subjects with PH-COPD on LTOT (IK-7002-COPD-201; NCT01728220). The primary outcome of this study was change in systolic pulmonary arterial pressure (sPAP) from baseline after treatment with iNO (measured by 2D transthoracic echocardiography with Doppler). The secondary outcome was the occurrence of a decrease ≥ 5 mm Hg of partial pressure of oxygen in arterial blood (PaO2) from baseline after treatment with iNO.

Subjects had a confirmed diagnosis of COPD by the Global initiative for chronic Obstructive Lung Disease (GOLD) criteria. Subjects also had tricuspid regurgitation velocity (TRV) ≥ 2.9 m/s as measured by echocardiogram, a post-bronchodilatory forced expiratory volume in 1 second/forced vital capacity (FEV1/FVC) < 0.7 and a FEV1 < 60% predicted. All subjects were at least 40 years old and were former smokers with at least 10 pack-years of tobacco cigarette smoking before study entry. All subjects also had been receiving LTOT for at least 3 months for at least 10 hours per day.

PH-COPD subjects were administered pulsed iNO at a dose of 3, 10, 15, 30 or 75 mcg/IBW kg/hr for at least 20 minutes, or received pulsed placebo (99.999% N₂) for at least 20 minutes. The doses of 3 and 10 mcg/kg IBW/hr were administered from a mini-cylinder having 2,440 ppm NO and the doses of 15, 30 and 75 mcg/kg IBW/hr were administered from a mini-cylinder having 4,880 ppm NO.

The results showed that the iNO dose of 3 mcg/kg IBW/hr (comparative dose) was ineffective, while the iNO doses of 10, 30 and 75 mcg/kg IBW/hr showed efficacy. The decrease in sPAP for all three doses was approximately the same. Surprisingly, the iNO dose of 15 mcg/kg IBW/hr did not show efficacy, although the lower dose of 10 mcg/kg IBW/hr and the higher dose of 30 mcg/kg IBW/hr did show efficacy.

The NO delivery device delivered gas comprising NO in a pulsatile manner at the beginning of the patient's breath. The minimum pulse volume is limited and for lower doses, can require 1 or more breaths to be skipped to maintain a constant dose in mcg/kg IBW/hr.

Assessment of the breath rate, dose and drug concentration revealed that the iNO dose of 15 mcg/kg IBW/hr had more breath skipping and a lower frequency of dosing compared to the iNO doses of 10 and 30 mcg/kg IBW/hr. Table 1 below shows the average sPAP, respiration rate and delivery frequency for the iNO doses of 10, 15, 30 and 75 mcg/kg IBW/hr.

**Table 1**

| | | | | |
|---|---|---|---|---|
| iNO Dose (mcg/kg IBW/hr) | 10 | 15 | 30 | 75 |
| Patients (N) | 21 | 21 | 24 | 24 |
| Average Change in sPAP vs Baseline | -4.4 | -2.4 | -4.5 | -4.3 |
| Average Respiration Rate during Treatment (bpm) | 17.9 | 19.2 | 17.3 | 17.8 |
| Average Skipped Breath | 1 | 2 | 1 | 0 |
| Average Delivery Frequency (seconds) | 6.7 | 9.4 | 6.9 | 3.4 |

As can be seen from Table 1, iNO dose of 15 mcg/kg IBW/hr averaged 2 skipped breaths, while the iNO doses of 10 and 30 mcg/kg IBW/hr averaged 1 skipped breath and the iNO dose of 75 mcg/kg IBW/hr typically did not skip breaths. In addition, the iNO dose of 15 mcg/kg IBW/hr delivered the pulse, on average, every 9.4 seconds (comparative time period), compared to the iNO doses of 10 and 30 mcg/kg IBW/hr that delivered at less than 7 seconds on average.

The data shows that increasing the time between pulses or increasing the number of skipped breaths will result in reduced or no efficacy of iNO therapy.

Reference throughout this specification to "one embodiment," "certain embodiments," "various embodiments," "one or more embodiments" or "an embodiment" means that a particular feature, structure, material, or characteristic described in connection with the embodiment is included in at least one embodiment of the disclosure. Thus, the appearances of the phrases such as "in one or more embodiments," "in certain embodiments," "in various embodiments," "in one embodiment" or "in an embodiment" in various places throughout this specification are not necessarily referring to the same embodiment of the disclosure. Furthermore, the particular features, structures, materials, or characteristics may be combined in any suitable manner in one or more embodiments.

The invention is defined by the appended claims.

## Claims

1. Nitric oxide for use in the treatment of pulmonary hypertension, the use comprising: administering a plurality of pulses of a gas comprising nitric oxide (NO) to a patient in need thereof over a plurality of breaths, wherein the gas comprising NO is not administered to the patient in at least one breath of the plurality of breaths, wherein the patient is administered a dose of NO in the range of about 5 to about 100 mcg/kg IBW/hr, and wherein:
(i) a maximum time period between successive pulses of the gas comprising NO does not exceed about 9 seconds, or
(ii) at least 400 pulses of the gas comprising NO is administered to the patient every hour.

2. Nitric oxide for use according to claim 1, wherein an average time period between successive pulses of the gas comprising NO does not exceed about 9 seconds; further optionally wherein an average time period between successive pulses of the gas comprising NO does not exceed about 7 seconds.

3. Nitric oxide for use according to claim 1 or 2, wherein at least 450 pulses of the gas comprising NO is administered to the patient every hour; optionally wherein at least 500 pulses of the gas comprising NO is administered to the patient every hour.

4. Nitric oxide for use according to any one of claims 1-3, wherein the patient has pulmonary arterial hypertension (WHO Group I).

5. Nitric oxide for use according to any one of claims 1-3, wherein the patient has WHO Group 3 pulmonary hypertension associated with interstitial lung disease (PH-ILD).

6. Nitric oxide for use according to any one of claims 1-3, wherein the patient has WHO Group 3 pulmonary hypertension associated with idiopathic pulmonary fibrosis (PH-IPF).

7. Nitric oxide for use according to any one of claims 1-3, wherein the patient has WHO Group 3 pulmonary hypertension associated with chronic obstructive pulmonary disease (PH-COPD).

8. Nitric oxide for use according to any one of claims 1-7 wherein the patient is administered a dose of NO in the range of about 30 to about 75 mcg/kg IBW/hr.

9. Nitric oxide for use according to any one of claims 1-8, wherein the gas comprising NO is administered for at least 15 minutes; optionally wherein the gas comprising NO is administered for at least at least 1 hour.

10. Nitric oxide for use according to any one of claims 1-9, wherein the gas comprising NO is administered for a plurality of days for at least 2 hours a day; optionally wherein the gas comprising NO is administered for a plurality of days for at least 6 hours a day.

11. Nitric oxide for use according to any one of claims 1-10, wherein the gas comprising NO is administered for at least 4 weeks.

12. Nitric oxide for use according to any one of claims 1-11, wherein the gas comprising NO is administered for at least 3 months.

## Patentansprüche

1. Stickstoffmonoxid zur Verwendung bei der Behandlung von pulmonaler Hypertonie, wobei die Verwendung umfasst: Verabreichen einer Vielzahl von Pulsen eines Stickstoffmonoxid (NO) umfassenden Gases an einen Patienten, der dessen bedarf, über eine Vielzahl von Atemzügen, wobei das NO umfassende Gas dem Patienten in mindestens einem Atemzug der Vielzahl von Atemzügen nicht verabreicht wird, wobei dem Patienten eine Dosis von NO im Bereich von etwa 5 bis etwa 100 mcg/kg IBW/h verabreicht wird, und wobei:
(i) eine maximale Zeitdauer zwischen aufeinanderfolgenden Pulsen des NO umfassenden Gases etwa 9 Sekunden nicht überschreitet, oder
(ii) dem Patienten mindestens 400 Pulse des NO umfassenden Gases jede Stunde verabreicht werden.

2. Stickstoffmonoxid zur Verwendung nach Anspruch 1, wobei eine mittlere Zeitdauer zwischen aufeinanderfolgenden Pulsen des NO umfassenden Gases etwa 9 Sekunden nicht überschreitet; ferner optional, wobei eine mittlere Zeitdauer zwischen aufeinanderfolgenden Pulsen des NO umfassenden Gases etwa 7 Sekunden nicht überschreitet.

3. Stickstoffmonoxid zur Verwendung nach Anspruch 1 oder 2, wobei dem Patienten mindestens 450 Pulse des NO umfassenden Gases jede Stunde verabreicht werden; optional, wobei dem Patienten mindestens 500 Pulse des NO umfassenden Gases jede Stunde verabreicht werden.

4. Stickstoffmonoxid zur Verwendung nach einem der Ansprüche 1-3, wobei der Patient pulmonal-arterielle Hypertonie (WHO-Gruppe I) hat.

5. Stickstoffmonoxid zur Verwendung nach einem der Ansprüche 1-3, wobei der Patient pulmonale Hypertonie der WHO-Gruppe 3 assoziiert mit interstitieller Lungenerkrankung (PH-ILD) hat.

6. Stickstoffmonoxid zur Verwendung nach einem der Ansprüche 1-3, wobei der Patient pulmonale Hypertonie der WHO-Gruppe 3 assoziiert mit idiopathischer Lungenfibrose (PH-IPF) hat.

7. Stickstoffmonoxid zur Verwendung nach einem der Ansprüche 1-3, wobei der Patient pulmonale Hypertonie der WHO-Gruppe 3 assoziiert mit chronisch obstruktiver Lungenerkrankung (PH-COPD) hat.

8. Stickstoffmonoxid zur Verwendung nach einem der Ansprüche 1-7, wobei dem Patienten eine Dosis von NO im Bereich von etwa 30 bis etwa 75 mcg/kg IBW/h verabreicht wird.

9. Stickstoffmonoxid zur Verwendung nach einem der Ansprüche 1-8, wobei das NO umfassende Gas für mindestens 15 Minuten verabreicht wird; optional, wobei das NO umfassende Gas für mindestens mindestens 1 Stunde verabreicht wird.

10. Stickstoffmonoxid zur Verwendung nach einem der Ansprüche 1-9, wobei das NO umfassende Gas für eine Vielzahl von Tagen für mindestens 2 Stunden pro Tag verabreicht wird; optional, wobei das NO umfassende Gas für eine Vielzahl von Tagen für mindestens 6 Stunden pro Tag verabreicht wird.

11. Stickstoffmonoxid zur Verwendung nach einem der Ansprüche 1-10, wobei das NO umfassende Gas für mindestens 4 Wochen verabreicht wird.

12. Stickstoffmonoxid zur Verwendung nach einem der Ansprüche 1-11, wobei das NO umfassende Gas für mindestens 3 Monate verabreicht wird.

## Revendications

1. Oxyde nitrique pour une utilisation dans le traitement de l'hypertension pulmonaire, l'utilisation comprenant : l'administration d'une pluralité d'impulsions d'un gaz comprenant de l'oxyde nitrique (NO) à un patient en ayant besoin sur une pluralité de respirations, dans lequel le gaz comprenant du NO n'est pas administré au patient lors d'au moins une respiration de la pluralité de respirations, dans lequel il est administré au patient une dose de NO comprise dans la plage d'environ 5 à environ 100 mcg/kg de IBW/h, et dans lequel :
(i) une période de temps maximale entre des impulsions successives du gaz comprenant du NO ne dépasse pas environ 9 secondes, ou
(ii) au moins 400 impulsions du gaz contenant du NO sont administrées au patient toutes les heures.

2. Oxyde nitrique pour une utilisation selon la revendication 1, dans lequel une période de temps moyenne entre des impulsions successives du gaz comprenant du NO ne dépasse pas environ 9 secondes ; en outre éventuellement dans lequel une période de temps moyenne entre des impulsions successives du gaz comprenant du NO ne dépasse pas environ 7 secondes.

3. Oxyde nitrique pour une utilisation selon la revendication 1 ou 2, dans lequel au moins 450 impulsions du gaz comprenant du NO sont administrées au patient toutes les heures ; éventuellement dans lequel au moins 500 impulsions du gaz comprenant du NO sont administrées au patient toutes les heures.

4. Oxyde nitrique pour une utilisation selon l'une quelconque des revendications 1 à 3, dans lequel le patient présente une hypertension artérielle pulmonaire (groupe I de l'OMS).

5. Oxyde nitrique pour une utilisation selon l'une quelconque des revendications 1 à 3, dans lequel le patient présente une hypertension pulmonaire du groupe 3 de l'OMS associée à une maladie pulmonaire interstitielle (HTP-MPI).

6. Oxyde nitrique pour une utilisation selon l'une quelconque des revendications 1 à 3, dans lequel le patient présente une hypertension pulmonaire du groupe 3 de l'OMS associée à une fibrose pulmonaire idiopathique (HTP-FPI).

7. Oxyde nitrique pour une utilisation selon l'une quelconque des revendications 1 à 3, dans lequel le patient présente une hypertension pulmonaire du groupe 3 de l'OMS associée à une maladie pulmonaire obstructive chronique (HTP-BPCO).

8. Oxyde nitrique pour une utilisation selon l'une quelconque des revendications 1 à 7, dans lequel il est administré au patient une dose de NO comprise entre environ 30 et environ 75 mcg/kg IBW/h.

9. Oxyde nitrique pour une utilisation selon l'une quelconque des revendications 1 à 8, dans lequel le gaz contenant du NO est administré pendant au moins 15 minutes ; éventuellement dans lequel le gaz comprenant du NO est administré pendant au moins 1 heure.

10. Oxyde nitrique pour une utilisation selon l'une quelconque des revendications 1 à 9, dans lequel le gaz comprenant du NO est administré pendant une pluralité de jours pendant au moins 2 heures par jour ; éventuellement dans lequel le gaz comprenant du NO est administré pendant une pluralité de jours pendant au moins 6 heures par jour.

11. Oxyde nitrique pour une utilisation selon l'une quelconque des revendications 1 à 10, dans lequel le gaz comprenant du NO est administré pendant au moins 4 semaines.

12. Oxyde nitrique pour une utilisation selon l'une quelconque des revendications 1 à 11, dans lequel le gaz comprenant du NO est administré pendant au moins 3 mois.
